# EUROPEAN PATENT APPLICATION

(11) **EP 4 353 261 A1**
(43) Date of publication of application: **17.04.2024**
(21) Application number: 22819693.7
(22) Date of filing: 09.06.2022
(51) Int. Cl.: A61K 45/00, A23L 33/00, A61P 25/00

(54) **NUTRACEUTIC COMBINATION AND ITS USE IN THE TREATMENT OF NEUROLOGICAL DISORDERS**

(30) Priority: 09.06.2021 ES 202130532
(71) Applicant: Hospital Sant Joan de Deu, 08950 Esplugues de Llobregat Barcelona (ES); Universitat de Barcelona, 08028 Barcelona (ES)
(72) Inventor: GARCÍA CAZORLA, Ángeles, 08950 Esplugues de Llobregat (ES); DE OYARZÁBAL SANZ, Alfonso Luis, 08950 Esplugues de Llobregat (ES); ALTAFAJ TARDÍO, Xavier, 08036 Barcelona (ES)
(74) Representative: ZBM Patents - Zea, Barlocci & Markvardsen
(86) International application number: PCT/ES2022/070361
(87) International publication number: WO 2022/258873

(57) **Abstract**

Disclosed is a nutraceutical combination comprising the following active ingredients: a) L-serine; b) a uridine derivative selected from the group formed by uridine monophosphate, a uridine phosphate, a uridine acid halide with a carboxylic acid (C₂-C₂₀), a uridine ester with a carboxylic acid (C₂-C₂₀), and a nutraceutical or pharmaceutically acceptable uridine salt; c) a compound selected from nicotinamide riboside, sulforaphane and mixtures thereof; and d) a compound selected from the group consisting of lecithin, choline, phosphatidylcholine, phosphatidylserine, N-acetylglucosamine, a polyunsaturated fatty acid (C₂₀-C₂₂), and mixtures thereof; as well as said combination for use in the treatment of neurodevelopmental disorders in children or adolescents, and products that contain the same.

## Description

This application claims the priority of Spanish patent application P202130532 filed on 9 June 2021.

### APPLICATION FIELD

The present invention relates to a nutraceutical combination comprising several compounds, products comprising same, as well as the combination for use in the treatment of neurological disorders, and in particular, in neuropediatric diseases.

### STATE OF THE ART

The term neurological disorder includes all those diseases that affect the central nervous system (the brain and spinal cord) and the peripheral nervous system (muscles and nerves). These disorders include epilepsy, Alzheimer's disease and other dementias, cerebrovascular diseases such as strokes, migraines and other headaches, multiple sclerosis, Parkinson's disease, neurological infections, brain tumors, traumatic conditions of the nervous system such as head injuries, and neurological disorders caused by malnutrition.

Neuropediatric diseases comprise a heterogeneous group of diseases that affect the developing brain, that is, the functions that the brain must develop in the period between 0 and 19 years. Similarly, they could be referred to as "neurodevelopmental diseases". Traditionally, and especially in Anglo-Saxon literature, "neurodevelopmental disorders" were linked almost exclusively to autism, attention deficit hyperactivity disorder (ADHD) and other learning difficulties. Nevertheless, this term includes without distinction the set of brain diseases occurring during the pediatric age, as neurodevelopmental diseases. These cover a broad spectrum of clinical phenotypes, not only those patients with autistic traits or learning difficulties, but all those symptoms of illness that a brain that is being formed and developed uses. This also includes intellectual disability, motor disorders such as "cerebral palsy" or other movement disorders (dyskinesias, ataxia, etc.) and epilepsy. In recent years, the term "developmental encephalopathy" has been introduced to indicate diseases of genetic origin that affect various functions of the developing brain at an early stage. In that sense, patients with these encephalopathies can present global difficulties in various areas: cognition, language, posture, muscle tone, motor functions such as the ability to sit autonomously, pick up objects, stand up, walk, etc. Neuropediatric diseases can occur due to specific mutations in particular genes or an unresolved etiology, and are distributed throughout the world.

In that sense, for example, an archetypal neurodevelopmental encephalopathy is Rett syndrome. It has an incidence of 1:10,000, but if more extensive entities of neuropediatrics such as autism and ASDs or intellectual disabilities are taken into account, estimates are between 0.4 and 3% incidence. Autism alone represents a health burden of 87.76 DALYs (Disability-Adjusted Life Years).

Rett syndrome is a congenital neurodevelopmental disorder that occurs primarily due to *de novo* mutations of the gene *MECP2* (located on the X chromosome and affecting mainly girls) and is the second most common cause of intellectual disability in girls. Patients with Rett syndrome present neurological regression, autism spectrum disorders, epilepsy and movement disorders. Despite multiple efforts to find treatments for Rett syndrome, there is currently no therapy available. The pathophysiological mechanisms underlying this disease are diverse and include abnormal bioenergetics, the alteration of neurotransmitters, inflammation and alterations of lipid metabolism.

Likewise, GRINpathies (GRDs) are a group of minority diseases, neurodevelopmental encephalopathies presenting with moderate/severe intellectual disability, communication deficits (related to the autism spectrum disorder), epilepsy and hypotonia. Genetically speaking, GRDs are the result of the presence of pathogenic variants of GRIN genes, which encode the subunits of NMDA-type glutamate receptors, the main components involved in the excitatory neurotransmission of the central nervous system.

Currently, the treatment of these diseases (autism spectrum disorders, epileptic encephalopathies, etc.) is mainly palliative, due to the lack of therapeutic alternatives that jointly correct neurochemical and neurometabolic deficits and are mostly directed at the management of symptoms and not the molecular basis of the disease.

Recently, some studies related to L-serine supplementation and its therapeutic benefit in improving the cognitive and motor function of patients suffering from neurodevelopmental encephalopathies have been published. Specifically, David Soto et al. in "L-Serine dietary supplementation is associated with clinical improvement of loss-of-function GRIN2B-related pediatric encephalopathy", Sci. Signal, 2019, vol. 12, pp. 1-15, explain that L-serine supplementation in the diet of a patient with mutations in the *GRIN2B* gene resulted in an increase in the abundance of plasma and brain D-serine, which is an amino acid also capable of activating NMDA-type glutamate receptors, without causing side effects. Receptors of this type are crucial for communication between nerve cells and play a fundamental role in learning mechanisms. According to this study, the patient noticed a considerable improvement in motor and cognitive function and communication after 11 and 17 months of supplementation.

Some nutritional supplements which combine several micronutrients to improve cognitive functions such as learning, memory and attention, as well as motor faculties, both in healthy individuals and in individuals with congenital or brain development disorders, are also known. Thus, for example, patent application WO2016083362A1 describes a composition comprising uridine, DHA and vitamin D3 for use as a nutritional supplement to improve cognitive functions.

Moreover, some combinations of nutrients have also been described for the treatment of other neurological diseases. In that sense, patent application WO2020/092616A1 describes a composition which may contain, among other ingredients, serine, choline, phosphatidylcholine, and nicotinamide riboside useful for the treatment of hyperhomocysteinemia, which is a disorder that has been linked to neurological conditions such as Alzheimer's disease, mild cognitive impairment, dementia and schizophrenia. However, these are different pathologies, specific to adults, and in the case of dementias, neurodegenerative disorders, with completely different pathophysiological pathways.

Nonetheless, most studies relating to the possible beneficial effect of some micronutrients alone or in combination with other micronutrients are aimed at improving cognitive function and little is known about how to improve the effects associated with muscular hypotonia in patients with disorders of this type, in particular difficulty or inability to walk, chew or swallow, that these patients present.

Therefore, from what is known in the state of the art it follows that there is still a need to provide a composition designed to improve several biological pathways that are altered in patients with neurodevelopmental diseases, improving their quality of life.

### DESCRIPTION OF THE INVENTION

The inventors have developed a combination of several compounds that is neuroprotective and allows modulating multiple biological pathways such as energy metabolism, glutamate neurotransmission or lipid metabolism, which are affected in children with neurodevelopmental disorders and other neuropediatric disorders, restoring them and improving their quality of life and contributing to the management of the disease, and also in adolescents. In that sense, the nutraceutical combination of the present invention allows acting on the molecular etiology of a broad spectrum of neuropediatric pathologies, without causing side effects.

The combination of the present invention provides the recipient with an improved health effect because there is an improvement in cognitive function accompanied by a considerable improvement in motor function. Being able to achieve an improvement in motor function is especially important in specific neurological disorders such as Rett syndrome and disorders related to GRIN genes, as well as in most early childhood encephalopathies.

Although some of the compounds included in the combination of the present invention have been tested alone, and even some of them in combination, as far as the inventors know, no combination that allows achieving the same neurological protection throughout development as the combination under study, let alone improves motor function to the level that the combination under study does has been described. Several of the compounds in the combination have never been linked as possible ingredients for use in the treatment thereof. The combination under study can be used in a broad spectrum of neurological disorders in children and adolescents that may benefit from improved cognitive and motor function.

In that sense, a first aspect of the present invention relates to a nutraceutical combination comprising at least the following active ingredients: a) L-serine; b) a uridine derivative selected from the group formed by uridine monophosphate, a uridine phosphate, a uridine acid halide with a carboxylic acid (C₂-C₂₀), a uridine ester with a carboxylic acid (C₂-C₂₀), and a nutraceutical or pharmaceutically acceptable uridine salt; c) a compound selected from nicotinamide riboside, sulforaphane, and mixtures thereof; and d) a compound selected from the group consisting of lecithin, choline, phosphatidylcholine, phosphatidylserine, N-acetylglucosamine, at least one polyunsaturated fatty acid (C₂₀-C₂₂) (PUFA) and mixtures thereof. As far as the inventors know, a nutraceutical combination with this combination of ingredients has not been previously described and it is considered from the tests carried out that this combination of components is mainly responsible for the beneficial effects observed in the formulation of the present invention.

The term "nutraceutical combination" as used herein refers to a concentrated combination of natural bioactive substances that has a greater favorable effect on health than what it could have when such substances are presented in foods at the usual doses. Likewise, the nutraceutical combination may also be able to provide medical benefits, including for the prevention and treatment of diseases. Furthermore, a nutraceutical combination can be administered as such in concentrated form, be added to a food to increase functional properties or administered in the form of a pharmaceutical product. The nutraceutical combination of the present invention is useful for providing neurological protection throughout development. Likewise, it is also suitable for improving motor function. The nutraceutical combination of the present invention is suitable for subjects in the stage of life in which there is melanogenesis, i.e., up to 25 years old, and in particular, for children and teenagers, and more particularly, in subjects aged between 0 and 19 years.

Melanogenesis is the process whereby the neuronal axon becomes covered with myelin, a substance that favors the transmission of nerve impulses. Thanks to melanogenesis, rapid transmission of nerve impulses between neurons is possible, promoting proper brain functioning. The nutraceutical combination of the present invention is suitable for treating neurodevelopmental (or neuropediatric) disorders where neurons may be underdeveloped, present alterations in bioenergetics or have defective neuronal communication mechanisms, since it favors cellular functions for the establishment of more efficient neuronal circuits.

The neurological disorders referred to in the invention are disorders of the developing brain, which are associated with young brains and do not include neurodegeneration, which presents with processes of neuronal death and progressive worsening, typical of adult pathologies and aging, as occurs in dementias and other related disorders.

L-serine is a nutraceutical amino acid that acts as an endogenous precursor of D-serine.

Uridine is a nucleoside that can be obtained from various food sources, such as sugar cane extract, tomatoes, beer, beets and broccoli.

The expression "uridine ester with a carboxylic acid (C₂-C₂₀)" refers to carboxylic acid esters derived from the corresponding carboxylic acid at the 5'C position of the ribose moiety. According to the invention, the alkyl chain of said carboxylic acid comprises from 2 to 20 carbon atoms. Examples of suitable carboxylic acids are acetic acid, fatty acids (myristic acid, stearic acid and oleic acid).

The expression "uridine acid halides with a carboxylic acid (C₂-C₂₀)" refers to derivatives of the corresponding carboxylic acid halide at the 5'C position of the ribose moiety. The alkyl chain of said acid halide comprises from 2 to 20 carbon atoms. Examples of suitable acid halides are acyl halides such as acyl chloride or fatty acid halides (of myristic acid, stearic acid and oleic acid).

The nutraceutical or pharmaceutically acceptable uridine salts should be non-toxic salts that are biologically tolerable or otherwise biologically suitable for administration to the subject. A nutraceutical or pharmaceutically acceptable salt is one that is pharmacologically effective and suitable for contact with the tissues of subjects without causing undue toxicity, irritation or allergic response.

Examples of uridine derivatives according to the invention include: uridine disodium diphosphate, uridine dipotassium phosphate, disodium uridine monophosphate, uridine diphosphate, uridine triphosphate, uridine triphosphate sodium, uridine triphosphate trisodium, uridine triphosphate trisodium trihydrate, triacetiyluridine, uridine diphosphoglucose, uridine diphosphogalactose, or uridine diphosphoglucosamine. In a particular embodiment, the nutraceutical combination of the present invention is one wherein the uridine derivative is uridine-5-monophosphate.

Nicotinamide riboside is a substance related to vitamin B3, found naturally in trace states in various foods such as milk.

Sulforaphane (SFN) is an isothiocyanate derived from glucoraphanin (GRA), found in large quantities especially in broccoli.

PUFAs are organic compounds formed by a long carbon chain attached to a carboxylic acid. The name comes from Poly-Unsaturated Fatty Acids. When the chain contains one or more double bonds between the carbons, it is said to be unsaturated and when it does not contain them it is saturated. Therefore, PUFAs are fatty acids that contain many double bonds in its carbon chain. When the nutraceutical combination of the present invention comprises polyunsaturated fatty acids, these have a carbon number between C₂₀-C₂₂. In a particular embodiment, the nutraceutical combination is one wherein the polyunsaturated fatty acid is selected from the group formed by docosahexaenoic acid (DHA), eicosapentaenoic acid (EPA), and mixtures thereof.

Docosahexaenoic acid (DHA; 22:6) is an omega-3 polyunsaturated fatty acid, more specifically, a carboxylic acid with a 22-carbon chain containing six cis double bonds. DHA is the main structural compound of the brain, the cerebral cortex, the skin, sperm, testes and retina. DHA can be isolated from a variety of sources. It can be obtained from fish, krill or algae. Cold water fish are particularly high in DHA. It can also be isolated from marine microalgae, for example, *Crypthecornium cohnii* and *Schizochytrium.* Some microalgae produce DHA.

Eicosapentaenoic acid (EPA; 20:5) is an omega 3 (ω-3), more specifically a carboxylic acid with a 20-carbon chain containing five cis double bonds. EPA is available in nuts, seeds and their oils, herring, mackerel, salmon, sardines and shad. It is also available from non-animal sources, such as supplements of spirulina and microalgae.

Where any compound mentioned herein may also exist as a nutraceutical or pharmaceutically acceptable salt, a solvate or a corresponding hydrate, the use of said salts, solvates or hydrates instead of or in addition to the mentioned compound is also part of the present invention.

In a particular embodiment, the nutraceutical combination of the present invention is one in which nicotinamide riboside and sulforaphane are present.

In a particular embodiment, the nutraceutical combination of the present invention comprises: a) L-serine; b) uridine-5-monophosphate; c) nicotinamide riboside, sulforaphane or mixtures thereof; and d) docosahexaenoic acid, eicosapentaenoic acid, or mixtures thereof.

In another particular embodiment, the nutraceutical combination of the present invention comprises: a) L-serine; b) uridine-5-monophosphate; c) nicotinamide riboside, d) sulforaphane; and e) docosahexaenoic acid, eicosapentaenoic acid, or mixtures thereof.

In another particular embodiment, the nutraceutical combination of the present invention comprises: L-serine; uridine-5-monophosphate, nicotinamide riboside, sulforaphane, docosahexaenoic acid and eicosapentaenoic acid.

In another particular embodiment in combination with any of the mentioned particular embodiments, the nutraceutical combination is one that further comprises spermidine. Spermidine, a common name for N'-(3-aminopropyl)butane-1,4-diamine, is a polyamine the structure of which consists of a aliphatic chain with three amino groups, two of them being primary, one at each end, and a secondary one.

In another particular embodiment in combination with any of the mentioned particular embodiments, the nutraceutical combination is one that further comprises creatine and vitamin B₆ (pyridoxine).

In another particular embodiment in combination with any of the mentioned particular embodiments, the nutraceutical combination is one that further comprises an active ingredient which is an amino acid selected from the group consisting of L-tyrosine, L-tryptophan, and mixtures thereof.

In another particular embodiment in combination with any of the mentioned particular embodiments, the nutraceutical combination is one that further comprises a compound selected from the group consisting of L-citrulline, L-arginine, and mixtures thereof.

In another particular embodiment in combination with any of the mentioned particular embodiments, the nutraceutical combination is one that further comprises a compound selected from the group consisting of selenium, magnesium, zinc, and mixtures thereof.

In another particular embodiment, the nutraceutical combination is one that comprises the following compounds: L-serine, uridine 5-monophosphate, nicotinamide riboside, sulforaphane, DHA, EPA, spermidine, creatine, and vitamin B₆.

In another particular embodiment, the nutraceutical combination is one that comprises the following compounds: L-serine, uridine 5-monophosphate, nicotinamide riboside, sulforaphane, DHA, EPA, spermidine, creatine, vitamin B₆, lecithin, choline, phosphatidylcholine, phosphatidylserine, N-acetylglucosamine, L-tyrosine, and L-tryptophan.

In another particular embodiment, the nutraceutical combination is one that comprises the following compounds: L-serine, uridine 5-monophosphate, nicotinamide riboside, sulforaphane, lecithin, choline, phosphatidylcholine, phosphatidylserine, N-acetylglucosamine, DHA, EPA, L-tyrosine, L-tryptophan, spermidine, L-citrulline, L-arginine, creatine, and vitamin B₆.

In another particular embodiment, the nutraceutical combination is one that comprises the following compounds: L-serine, uridine 5-monophosphate, nicotinamide riboside, sulforaphane, lecithin, choline, phosphatidylcholine, phosphatidylserine, N-acetylglucosamine, DHA, EPA, L-tyrosine, L-tryptophan, spermidine, L-citrulline, L-arginine, creatine, vitamin B₆, L-ornithine, selenium, magnesium and zinc.

In a particular embodiment, the nutraceutical combination is one that comprises an amount of each of the compounds that are present in the combination suitable for administering, in one or more daily doses, a total daily amount of each of the compounds present as follows: L-serine at a daily dose of 200 to 800 mg/kg/day; uridine derivative at a daily dose of 500 to 2000 mg/20kg/day, in particular, uridine-5-monophosphate; nicotinamide riboside at a daily dose of 450 to 1800 mg/20kg/day and/or sulforaphane at a daily dose of 4 mg/20kg/day to 16 mg/20kg/day; at least one polyunsaturated fatty acid (C₂₀-C₂₂) at a daily dose of 105 to 420 mg/kg/day; lecithin at a daily dose of 500 to 2000 mg/20kg/day; choline at a daily dose of 250 to 1000 mg/20kg/day, phosphatidylcholine at a daily dose of 450 to 1800 mg/20kg/day; phosphatidylserine at a daily dose of 150 to 600 mg/20kg/day; and N-acetylglucosamine at a daily dose of 450 to 1800 mg/20kg/day.

In a particular embodiment, the at least one polyunsaturated fatty acid (C₂₀-C₂₂) is present and is a mixture of DHA and EPA, wherein DHA is at a daily dose of 45 to 180 mg/kg/day; and EPA is at a dose of 60 to 240 mg/kg/day. In another particular embodiment, DHA is at a daily dose of 80 to 100 mg/kg/day; and EPA is at a dose of 110 to 130 mg/kg/day.

In another particular embodiment, the nutraceutical combination of the present invention is one that comprises the following compounds that are administered at a daily dose as indicated below: L-serine at a dose of 300 to 500 mg/kg/day; uridine derivative at a dose of 900 to 1100 mg/20kg/day; nicotinamide riboside at a dose of 800 to 1000 mg/20kg/day; DHA at a dose of 80 to 100 mg/kg/day; and EPA at a dose of 110 to 130 mg/kg/day.

The nutraceutical combinations of the present invention may comprise other additional compounds; where one or more of them are present, they are present in an amount suitable for administering, in one or more daily doses, a total daily amount of each of the compounds present as follows: spermidine at a daily dose of 2 mg/20kg/day to 16 mg/20kg/day; L-tyrosine at a daily dose of 75 to 300 mg/kg/day; L-tryptophan at a daily dose of 50 to 200 mg/20kg/day; L-citrulline at a daily dose of 50 to 200 mg/kg/day; L-arginine at a daily dose of 50 to 200 mg/kg/day; creatine at a dose of 150 to 600 mg/kg/day; vitamin B₆ at a daily dose of 30 to 120 mg/20kg/day; selenium at a daily dose of 0.05 to 0.2 mg/20kg/day; magnesium at a daily dose of 15 to 60 mg/20kg/day; and zinc at a daily dose of 2.5 to 10 mg/20kg/day.

In another particular embodiment, the nutraceutical combination of the present invention comprises an amount of L-serine suitable for administering, in one or more daily doses, a daily dose of 300 to 500 mg/kg/day. In another particular embodiment, the nutraceutical combination comprises an amount of a uridine derivative suitable for administering, in one or more daily doses, one at a daily dose of 900 to 1100 mg/20kg/day. In another particular embodiment, the nutraceutical combination comprises nicotinamide riboside at a daily dose of 800 to 1000 mg/20kg/day. In another particular embodiment, the nutraceutical combination comprises an amount of DHA suitable for administering, in one or more daily doses, a daily dose of 80 to 100 mg/kg/day; and EPA at a daily dose of 110 to 130 mg/kg/day. In another particular embodiment, the nutraceutical combination comprises an amount of sulforaphane suitable for administering, in one or more daily doses, a daily dose of 6 mg/20kg/day to 10 mg/20kg/day. In another particular embodiment, the nutraceutical combination comprises an amount of lecithin suitable for administering, in one or more daily doses, a daily dose of 800 to 1100 mg/20kg/day. In another particular embodiment, the nutraceutical combination comprises an amount of choline suitable for administering, in one or more daily doses, a daily dose of 400 to 600 mg/20kg/day. In another particular embodiment, the nutraceutical combination comprises an amount of phosphatidylcholine suitable for administering, in one or more daily doses, a daily dose of 800 to 1000 mg/20kg/day. In another particular embodiment, the nutraceutical combination comprises an amount of phosphatidylserine suitable for administering, in one or more daily doses, a daily dose of 200 to 400 mg/20kg/day. In another particular embodiment, the nutraceutical combination comprises an amount of N-acetylglucosamine suitable for administering, in one or more daily doses, a daily dose of 800 to 1000 mg/20kg/day. In another particular embodiment, the nutraceutical combination comprises an amount of spermidine suitable for administering, in one or more daily doses, a daily dose of 2 mg/day to 6 mg/20kg/day. In another particular embodiment, the nutraceutical combination comprises an amount of L-tyrosine suitable for administering, in one or more daily doses, a daily dose of 100 to 200 mg/kg/day. In another particular embodiment, the nutraceutical combination comprises an amount of L-tryptophan for administering, in one or more daily doses, a daily dose of 75 to 125 mg/20kg/day. In another particular embodiment, the nutraceutical combination comprises an amount of L-citrulline suitable for administering, in one or more daily doses, a daily dose of 75 to 125 mg/kg/day. In another particular embodiment, the nutraceutical combination comprises an amount of L-arginine suitable for administering, in one or more daily doses, a daily dose of 75 to 125 mg/kg/day. In another particular embodiment, the nutraceutical combination comprises an amount of creatine suitable for administering, in one or more daily doses, a daily dose of 200 to 400 mg/kg/day. In another particular embodiment, the nutraceutical combination comprises an amount of vitamin B₆ suitable for administering, in one or more daily doses, a daily dose of 40 to 80 mg/20kg/day. In another particular embodiment, the nutraceutical combination comprises an amount of selenium suitable for administering, in one or more daily doses, a daily dose of 0.05 to 0.15 mg/20kg/day. In another particular embodiment, the nutraceutical combination comprises an amount of magnesium suitable for administering, in one or more daily doses, a daily dose of 25 to 35 mg/20kg/day. In another particular embodiment, the nutraceutical combination comprises an amount of zinc suitable for administering, in one or more daily doses, a daily dose of 4 to 6 mg/20kg/day.

In another particular embodiment, the nutraceutical combination of the present invention is one that comprises the following compounds that are administered at a daily dose as indicated below: L-serine at a dose of 400 mg/kg/day; uridine derivative, in particular, uridine-5-monophosphate, at a dose of 1000 mg/20kg/day; nicotinamide riboside at a dose of 900 mg/20kg/day; DHA at a dose of 90 mg/kg/day; and EPA at a dose of 120 mg/kg/day.

In another particular embodiment, the following additional compounds, if present, are administered at a daily dose as follows: sulforaphane at a daily dose of 8 mg/20kg/day; lecithin at a daily dose of 1000 mg/20kg/day, choline at a daily dose of 500 mg/20kg/day, phosphatidylcholine at a daily dose of 900 mg/20kg/day, phosphatidylserine at a daily dose of 300 mg/20kg/day, N-acetylglucosamine at a daily dose of 900 mg/20kg/day, spermidine at a daily dose of 4 mg/20kg/day, L-tyrosine at a daily dose of 150 mg/kg/day, L-tryptophan at a daily dose of 100 mg/20kg/day, L-citrulline at a daily dose of 100 mg/kg/day, L-arginine at a daily dose of 100 mg/kg/day, creatine at a daily dose of 300 mg/kg/day, vitamin B₆ at a daily dose of 60 mg/20kg/day, selenium at a daily dose of 0.1 mg/20kg/day, magnesium at a daily dose of 30 mg/20kg/day, zinc at a daily dose of 5 mg/20kg/day.

Any combination of these embodiments with other embodiments described herein also form part of the invention. For the purposes of the invention, indicated ranges include both the lower and upper end points of the indicated range.

To provide a more concise description, some of the quantitative expressions given here are not qualified with the term "approximately". It is understood that whether the term "approximately" is used explicitly or not, each amount given here refers to the real value given, and also to the approximation to said value that could reasonably be inferred by a person skilled in the art, including equivalents and approximations due to the experimental and/or measurement conditions of said given value. The description that refers to "about X" includes the description of "X".

The nutraceutical combinations of the present invention can be prepared by conventional methods, specifically, they can be prepared by mixing the components in any order. In a particular embodiment, the method for preparing the nutraceutical combination comprises mixing solid ingredients on one hand, and liquid ingredients on the other.

The nutraceutical combination of the present invention can be administered to the patient in various forms.

Thus, one aspect of the present invention relates to a preparation in liquid or suspension form comprising both the solid and liquid compounds of the nutraceutical combination according to the present invention. Optionally, this preparation comprises nutraceutical or pharmaceutically acceptable excipients or carriers. This preparation is a nutraceutical preparation.

In a particular embodiment, this preparation can be on a stick, for example, polyester/aluminum/polyethylene. The stick format refers to a single-dose container that is shaped like an elongated tube that is easy to open and has the advantage of guaranteeing precise dosage of the dose to be administered. The preparation in liquid or suspension form present in the stick is suitable to be taken directly from the stick or with water. It could also be administered mixed, for example, with food.

Also part of the invention is a preparation in powder form comprising a nutraceutical combination as defined above, wherein the compounds thereof are solid. This preparation can be presented, for example, in the form of sachets.

The above preparations comprise a nutraceutically effective amount of the combination of the present invention. A nutraceutically effective amount is one that has a greater favorable effect on health than it could have when such substances are present in food in the usual doses and that improves the state of health and well-being.

Also part of the invention is a dosage form that is a combination of powders that are dissolved in a drink or food such as (water/milk/yogurt, etc.) with beads or with a syrup that contains part of the components. Also part of the invention is a combination of powders with a gummy that contains part of the components.

Also part of the present invention is a pharmaceutical product comprising a therapeutically effective amount of the nutraceutical combination defined above, optionally, with pharmaceutically acceptable excipients or carriers. The pharmaceutical product of the present invention can be presented in stick, sachet, tablet or capsule format or combinations thereof. The stick format is suitable for pharmaceutical products that are in liquid or suspension form, i.e., those that comprise solid and liquid components of the combination of the invention. Optionally, these comprise pharmaceutically acceptable excipients or carriers. The sachet, tablet or capsule format is suitable for pharmaceutical products in which the components of the nutraceutical combination are all in solid form.

The term "therapeutically effective amount", as used herein, refers to the amount of a nutraceutical combination which, when administered, is sufficient to prevent the development of, or to alleviate to some extent, one or more of the symptoms of the disease addressed. The particular dose of the nutraceutical combination administered according to this invention will be determined, of course, by the particular circumstances of the case, including the particular condition being treated, and similar considerations.

The expression "pharmaceutically acceptable excipients or carriers" refers to pharmaceutically acceptable materials, compositions or carriers, respectively. Each component must be pharmaceutically acceptable in the sense of being compatible with the other ingredients of the nutraceutical or pharmaceutical combination. It must also be suitable for use in contact with human and animal tissues or organs without excessive toxicity, irritation, allergic response, immunogenicity or other problems or complications consistent with a reasonable risk/benefit ratio.

The compounds of the nutraceutical combination may also be provided in a kit. In that sense, also part of the invention is a kit comprising two or more components, wherein each component comprises one or more of the compounds of the nutraceutical combination defined above, wherein when the kit comprises one or more compounds in liquid form, they are either in separate liquid components or together in a single liquid component. For example, polyunsaturated fatty acids such as DHA or EPA, phosphatidylcholine and phosphatidylserine would be part, if present, of the liquid component. The components of the nutraceutical combination are the following: a) L-serine; b) a uridine derivative selected from the group formed by uridine monophosphate, a uridine phosphate, a uridine acid halide with a carboxylic acid (C₂-C₂₀), a uridine ester with a carboxylic acid (C₂-C₂₀), and a nutraceutical or pharmaceutically acceptable uridine salt; c) nicotinamide riboside, sulforaphane, or mixtures thereof; d) a compound selected from the group consisting of lecithin, choline, phosphatidylcholine, phosphatidylserine, N-acetylglucosamine, at least one polyunsaturated fatty acid (C₂₀-C₂₂), and mixtures thereof; and e) optionally one or more compounds selected from the group consisting of: spermidine; creatine and vitamin B₆; an active ingredient selected from the group consisting of L-tyrosine, L-tryptophan, and mixtures thereof; a compound selected from the group consisting of L-citrulline, L-arginine, and mixtures thereof, and a compound selected from the group consisting of selenium, magnesium, zinc, and mixtures thereof. The components that would be part, if present, of the liquid component are selected from the group consisting of polyunsaturated fatty acids, phosphatidylcholine and phosphatidylserine.

In a particular embodiment, the kit is one that comprises: a) a first component comprising the compounds of the combination that are solid; and b) a second component comprising the compounds that are in liquid form.

In a particular embodiment, the first component comprising the compounds of the combination that are solid is the preparation in powder form defined above. In another particular embodiment, the kit of the present invention is a pharmaceutical kit in which the first component comprising the compounds of the combination that are solid is the pharmaceutical product defined above in which the components are solid. In the event that the nutraceutical combination comprises liquid compounds, these are administered simultaneously or sequentially with the compounds of the combination that are solid.

When reference is made to solid compounds, they are compounds that are solid at room temperature (T= 20-25°C) and when reference is made to liquid compounds, they are compounds that are liquid at room temperature or solid compounds that are sold in liquid format.

The kit allows the doses of each of the compounds to be adjusted according to the patient's kg, particularly in the case of children at an early age (0-6 years), but mainly for children from 0 to 3 years old. Generally, from 20 kg in weight, a standard daily dose can be administered.

Another aspect of the present invention relates to a food product in powder form comprising those nutraceutical combinations of the present invention, wherein the compounds thereof are solid. In the event that the nutraceutical combination comprises liquid compounds, these are administered simultaneously or sequentially with the food product in the form of a liquid preparation. The amount of the nutraceutical combination in the food is that which allows the recommended daily dose of each of the compounds present in the nutraceutical combination to be administered.

In a particular embodiment, the food product is an infant formula in powder form that is intended to be mixed with water before use or a ready-to-use liquid product. The term "ready-to-use product", as used herein, refers to liquid formulations suitable for direct oral administration to a child, in which the formulas are ready-to-feed liquids, reconstituted powders or diluted concentrates.

The pharmaceutical compositions or food products of the present invention may be prepared by conventional methods.

Another aspect of the present invention is a nutraceutical combination as defined above for use in the treatment of neurological disorders in children or adolescents. This aspect may also be formulated as the use of a nutraceutical combination as defined above for the manufacture of a nutraceutical product or a medicament for the treatment of neurological disorders in children or adolescents. The present invention also relates to a method for the treatment of neurological disorders, comprising the administration of a therapeutically effective amount of the nutraceutical combination as defined above to a subject in need thereof, where said subject is a child or an adolescent. These neurological disorders in children and adolescents are referred to as neuropediatric diseases, that is, with repercussions on neurodevelopment. These diseases present with neurobiological alterations in the brain affecting the main functions that normally guarantee correct brain development. This involves alterations in the size and shape of neurons, number of synaptic boutons/spines, connections that neurons establish between themselves, energy dynamics, formation and maturation of myelin and other neuronal lipids, as well as other aspects of metabolism, producing a cognitive and/or motor function condition. The affected child or adolescent suffers from brain disorders, often of genetic origin, that cause a disruption in the fundamental mechanisms of brain development. These disorders appear in stages of neurodevelopment in which there is active melanogenesis. The stage of life in which there is active melanogenesis can last up to 25 years.

In particular, the combination for use as defined above is for a neurodevelopmental disorder in children or adolescents (0 to 25 years). These neurologically based neurodevelopmental disorders can affect the acquisition, retention or application of specific skills or sets of information, and consist of alterations in attention, memory, perception, language, problem solving or social interaction.

In another particular embodiment, the combination for use as defined above is for a neurodevelopmental disorder in children. Specifically, in a particular embodiment, the combination of the present invention can be used in children (0 to 10 years old) and adolescents (11 to 19 years old), and it is also especially useful for early ages (between 0 and 6 years of age).

In another particular embodiment, the subject is a subject suffering from congenital or developmental brain disorders. In another particular embodiment, the affected population has autistic traits, intellectual disability or learning difficulties. In another particular embodiment, the neurodevelopmental disorder is selected from the group consisting of Rett syndrome and GRINpathies (GRDs). Grinpathies include a set of rare diseases due to pathogenic mutations that occur in one of the genes called GRIN (GRIN1, 2A, 2B, 2C, 2D, 3A or 3B). The effects vary in each case, and include, to a greater or lesser extent, moderate/severe intellectual disability, communication deficits (related to the autism spectrum disorder), epilepsy and muscular hypotonia (difficulty or inability to walk, chew or swallow, etc.). In another particular embodiment, the combination for use as defined above is one in which the neurodevelopmental disorder is a GRIN2B-related pediatric encephalopathy.

In another particular embodiment, the combination for use as defined above is one in which the treatment comprises improving psychomotor function. In another particular embodiment, the combination for use as defined above is one in which the treatment comprises improving cognitive function. In another particular embodiment, the combination for use as defined above is one in which the treatment comprises improving motor function and cognitive function.

Also forming part of the present invention is the use of the combination as defined above to improve cognitive functions and/or motor functions in a subject in need thereof. The subject may be, for example, a healthy subject or a subject who does not suffer from any neurodegenerative disease but who has motor function problems. This use comprises administering to the subject an effective amount of the nutraceutical combination of the present invention. In a particular embodiment, the use comprises improving learning in individuals with developmental coordination disorders. In another embodiment, the use comprises improving memory in individuals with developmental coordination disorder.

All particular embodiments defined above for the nutraceutical combination, in particular in relation to the daily doses of the components, are also particular embodiments of the uses indicated in the present invention.

For children weighing less than 20 kg, generally children from 0 to 3 years old, daily doses must be adapted to the child's weight. From 20 kg, the doses can be considered standard. To do this, the target population is divided into four groups: infants, preschool (up to 6 years or 20 kg), school and adolescent. In the first two groups (up to 6 years), doses will be adapted to the patient's weight. In the two older groups, it will be standardized in 10 kg intervals.

The nutraceutical combination of the present invention is for oral administration. Generally, the recommended daily dose is administered divided into three doses a day.

Throughout the description and the claims, the word "comprises" and its variants do not intend to exclude other technical features, additives, components or steps. Furthermore, the word "comprises" includes the case of "consists of". For those skilled in the art, other objects, advantages and features of the invention may be partially deduced from both the description and the embodiment of the invention. The following examples and drawings are provided by way of illustration and are not intended to limit the present invention. The numerical signs relating to the drawings and placed in parentheses in a claim are only intended to try to increase understanding of the claim and should not be interpreted as limiting of the scope of protection of the claim. Furthermore, the present invention covers all possible combinations of particular and preferred embodiments indicated herein.

### BRIEF DESCRIPTION OF THE FIGURES

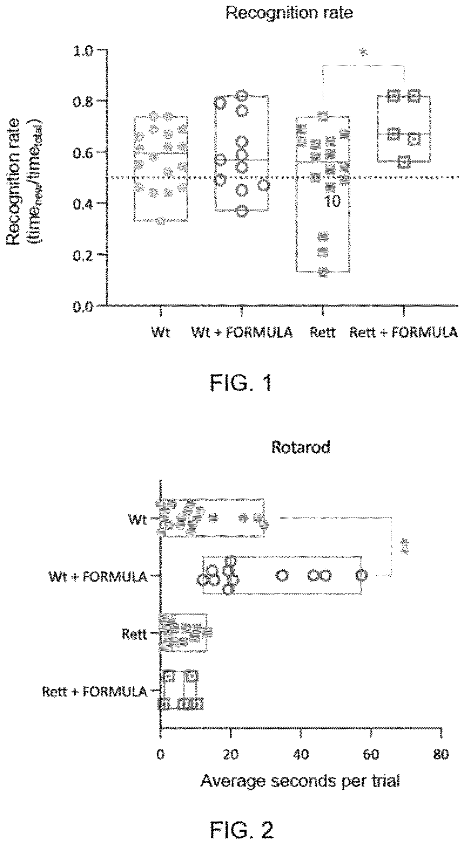

Figure 1 shows the results of NORT tests in animal models of Rett syndrome.
Figure 2 shows the results of Rotarod tests in animal models of Rett syndrome.
Figure 3 shows the results of a trial with patients in which the following aspects are evaluated: behavior, attention, stereotypes, language, motor skills and socialization.

### EXAMPLES

### Example 1: Preparation of a formulation according to the present invention

The tested formula is described in the following table. It is formed by the combination of several nutraceutical products. The amount of each product is established as the amount intended to be given to patients (humans) (mg of product/20kg/day), as indicated in (Table 1, column 2).

In the proof-of-concept experiments, these amounts were extrapolated to the mean weight of a C57BL/6J mouse (30g), with the results indicated in Table 1, column 3.

To facilitate administration, the compounds were mixed with the normal diet in the form of standard pellets, leaving the mice free access to the food. According to Bachmanov *et al.,* 2002, a C57BU6J mouse has a mean daily food intake of 5 g. Therefore, the products were mixed at a concentration such that each daily need for the product was present per 5 g/total food.

For example: the dose for lecithin is 1 g/20kg/day in patients. Therefore, 1.5 mg/mouse/day was calculated. In the final mixture, there would be 1.5 mg of lecithin/5g of total mixture (i.e. 300 mg of lecithin/kg of mixture) (see Table 1, column 4)

**Table 1:**

| | PATIENTS | MICE | CONTENT IN MICE DIET |
|---|---|---|---|
| | | (mg/30g/day) | (mg/kg of mixture) |
| Lecithin | 1000 mg/20kg/day | 1.5 | 300 |
| Choline | 500 mg/20kg/day | 0.75 | 150 |
| Phosphatidylcholine | 900 mg/20kg/day | 1.35 | 464.3 |
| Phosphatidylserine | 300 mg/20kg/day | 0.45 | 450 |
| N-acetylglucosamine | 900 mg/20kg/day | 1.35 | 270 |
| DHA | 90 mg/kg/day | 2.7 | 4900 |
| EPA | 120 mg/kg/day | 3.6 | |
| L-serine | 400 mg/kg/day | 12 | |
| L-citrulline | 100 mg/kg/day | 3 | |
| L-arginine | 100 mg/kg/day | 3 | 600 |
| L-tyrosine | 150 mg/kg/day | 4.5 | 900 |
| L-tryptophan | 100 mg/20kg/day | 0.15 | 30 |
| Uridine 5-monophosphate | 1000 mg/20kg/day | 1.5 | 300 |
| Sulforaphane | 8 mg/20kg/day | 0.013 | 2.65935 |
| Nicotinamide riboside | 900 mg/20kg/day | 1.35 | 270 |
| Creatine | 300 mg/kg/day | 9 | 1800 |
| Vitamin B₆ | 60 mg/20kg/day | 0.09 | 18 |
| Selenium | 0.1 mg/20kg/day | 0.0002 | 0.03 |
| Magnesium | 30 mg/20kg/day | 0.045 | 9 |
| Zinc | 5 mg/20kg/day | 0.0075 | 1.5 |

The same diet without compounds was given to the control groups.

All compounds, except the following, were obtained in pure presentations. Phosphatidylcholine and phosphatidylserine were in presentations of 36 and 20% purity a/a (measured by HPLC), and EPA and DHA were present in a fluid mixture at 160 and 110 mg/g, respectively. These concentrations were taken into account when preparing the final mixture at the concentrations indicated in Table 1, column 3.

### Example 2: Nutraceutical combinations according to the present invention

### Other formulations of the present invention are included in this example:

### Nutraceutical combination A:

| | PATIENTS |
|---|---|
| DHA | 90 mg/kg/day |
| EPA | 120 mg/kg/day |
| L-serine | 400 mg/kg/day |
| Uridine 5-monophosphate | 1000 mg/20kg/day |
| Nicotinamide riboside | 900 mg/20kg/day |
| Spermidine | 4 mg/20kg/day |
| Creatine | 300 mg/kg/day |
| Vitamin B₆ | 60 mg/20kg/day |

### Nutraceutical combination B

| | PATIENTS |
|---|---|
| DHA | 90 mg/kg/day |
| EPA | 120 mg/kg/day |
| L-serine | 400 mg/kg/day |
| Uridine 5-monophosphate | 1000 mg/20kg/day |
| Nicotinamide riboside | 900 mg/20kg/day |
| Sulforaphane | 8 mg/20kg/day |
| Spermidine | 4 mg/20kg/day |
| Creatine | 300 mg/kg/day |
| Vitamin B₆ | 60 mg/20kg/day |

### Nutraceutical combination C

| | PATIENTS |
|---|---|
| DHA | 90 mg/kg/day |
| EPA | 120 mg/kg/day |
| Phosphatidylcholine | 900 mg/20kg/day |
| Phosphatidylserine | 300 mg/20kg/day |
| L-serine | 400 mg/kg/day |
| Uridine 5-monophosphate | 1000 mg/20kg/day |
| Nicotinamide riboside | 900 mg/20kg/day |
| Sulforaphane | 8 mg/20kg/day |
| Spermidine | 4 mg/20kg/day |
| Creatine | 300 mg/kg/day |
| Vitamin B₆ | 60 mg/20kg/day |

### Nutraceutical combination D

| | PATIENTS |
|---|---|
| Lecithin | 1000 mg/20kg/day |
| Choline | 500 mg/20kg/day |
| Phosphatidylcholine | 900 mg/20kg/day |
| Phosphatidylserine | 300 mg/20kg/day |
| N-acetylglucosamine | 900 mg/20kg/day |
| DHA | 90 mg/kg/day |
| EPA | 120 mg/kg/day |
| L-serine | 400 mg/kg/day |
| L-citrulline | 100 mg/kg/day |
| L-arginine | 100 mg/kg/day |
| L-tyrosine | 150 mg/kg/day |
| L-tryptophan | 100 mg/20kg/day |
| Uridine 5-monophosphate | 1000 mg/20kg/day |
| Spermidine | 4 mg/20kg/day |
| Sulforaphane | 8 mg/20kg/day |
| Nicotinamide riboside | 900 mg/20kg/day |
| Creatine | 300 mg/kg/day |
| Vitamin B₆ | 60 mg/20kg/day |
| Selenium | 0.1 mg/20kg/day |
| Magnesium | 30 mg/20kg/day |
| Zinc | 5 mg/20kg/day |

### Example 3: Evaluation of the formulation of example 1 mouse model of Rett disease

The formulation was tested in a mouse model of Rett disease. The Mecp2tm1.1 Bird strain, which is widely accepted by the scientific community as a reliable model for Rett syndrome, was selected. This strain has a C57BL/6J genetic background and carries a null mutation (knockout) of Mecp2, deleting exons 3 and 4 (Guy, J., Hendrich, B., Holmes, M., Martin, J. E. & Bird, A. A mouse Mecp2-null mutation causes neurological symptoms that mimic Rett syndrome. Nat Genet 27, 322-326 (2001)).

Females from a colony that began with 10 females several years ago, acquired from *The Jackson Laboratory,* have been used. Heterozygous Mecp2 females have been crossed with C57BL/6J males in each generation.

Females have been used because they recapitulate the disease better. While affected males show premature onset and die within the first two months, females in this model show normal early postnatal development, presenting complete symptoms around 6 months of age.

Four experimental groups were established: two treated groups (*wt* mice and Rett) and two control groups (both *wt* mice and Rett). Littermates were used in all four groups to minimize possible differences recorded from different genetic backgrounds.

The treated groups were provided with a diet *ad libitum* that contained the neuroprotective formula at the concentrations explained above, from weaning to sacrifice. The control groups were given the same diet, but without any formula. One week before the animals were sacrificed, two behavioral tests were performed. The two behavioral tests were:
a) NORT (Novel Object Recognition Test): Widely used as a tool to investigate recognition memory, it is based on the innate exploratory behavior of rodents. The experiment takes place on two consecutive days.

In summary, in the experiment, on the first day, two identical figures are presented to the animal (in this case two plastic figures or two chess pieces). The mouse is left on a cube(*) with both figures at an equal distance and left to explore for 10 minutes. Then, the mouse is returned to its cage until the next day.

The second day, one of the figures is changed (randomly in each animal) and is presented like on the previous day. The exploration time of each figure is recorded up to a maximum exploration time of 20s or 10' in total. The recognition rate is calculated in each experiment as the time spent exploring the new object with respect to the total exploration time. If the mouse did not reach a total exploration time of 8", the subject was discarded from the experiment. A recognition rate of 0.5 suggests poor recognition memory, since the animal does not discriminate between both objects; higher recognition rates suggest better memory.

^{(*)}Technical notes on the experimental setup: The cube has a dimension of 35 x 35 cm. The pieces presented were different in appearance, but made of plastic and of similar texture and size, so this did not bias the experiment. The entire setup and figures were cleaned after each experiment, so there were no residual odors.

b) ROTAROD: This is a performance test based on the application of a rotating rod to the mouse, measuring driving time and thus evaluating balance, grip strength and motor coordination. The animal was placed rear-facing on a rotating cylinder (rod) oriented horizontally and suspended above the floor of the cage. The rod rotated at a speed of 4 rpm, increased to 40 rpm in five minutes. The seconds until the mouse fell were measured. Three consecutive tests were carried out for each animal, with a period of one minute between them.

^{(*)}Technical notes on the experimental setup: For this test, a "ROTA ROD" apparatus from *Panlab Harvard Apparatus* was used.

### RESULTS:

The aforementioned tests were carried out in the four experimental groups: wt (n=16), Rett (n=15), wt-treated (n=11) and Rett-treated (n=7).

The analysis of the Novel Object Recognition Test revealed a significant increase in the recognition rate in Rett mice with the neuroprotective treatment. The recognition rate increased from 0.53 in the case of Rett to 0.704 in the treated case with Rett. This difference was statistically significant, applying a Brown-Forsythe and Welch ANOVA test (distribution of normality was previously checked).

Regarding the analysis of motor coordination and balance through the Rotarod test, a significant increase in motor performance was recorded in control mice treated with the neuroprotective diet. It should be taken into account that Rett heterozygous females develop atrophy of the legs from three months of age, making it difficult to walk and, obviously, to grip. This represents a physical impediment to carrying out the test, evidenced by their poor performance, both treated and untreated. However, the significant observation in *wt* mice indicates that a similar increase in motor coordination can be expected in those patients in whom the anatomical impairment is not particularly pronounced.

### Example 4: Evaluation of various combinations with the main compounds of the formulation in patients

Several combinations of different elements have been tested and are considered to be mainly responsible for the formulation of Example 1 leading to an improvement not only in behavior and attention, but also a remarkable improvement in the improvement of motor function of the patients tested. The patients suffered from developmental disorders leading to hypoglutamatergic transmission.

Improvement in motor ability has been observed in all patients (humans), followed by improvements in behavior, attention and learning.

Table 2 summarizes the tested combinations, the diagnosis, the treatment time and the observed effect. The numbers in parentheses in the "evolution" column mean the following: 0: no improvement; 1: slight improvement; 2: moderate improvement; 3: significant improvement.

The administered amounts of each of the compounds are those indicated in Table 1 of Example 1 for patients, i.e., humans. For example, where Table 2 indicates that L-serine+5HT is administered, this combination comprises the amounts of these components indicated in Table 1, which are 400 mg/kg/day of L-serine and 1000 mg/20kg/day of 5HT. Where it indicates that L-serine+5HTP+DHA is administered, this combination comprises the amounts of these components indicated in Table 1, which are 400 mg/kg/day of L-serine, 1000 mg/20kg/day of 5HTP and 90 mg/kg/day of DHA. Where it indicates that Nicotinamide+DHA is administered, this combination comprises the amounts of these components indicated in Table 1, which are 900 mg/20kg/day of nicotinamide riboside and 90 mg/kg/day of DHA.

**Table 2**

| | Patient | Diagnosis | Treatment time | Evolution |
|---|---|---|---|---|
| L-serine+5-HT | | | | |
| | Patient 1 | TELO2 | 6 months | Behavior (1) + Attention (1) + Motor (2) |
| | Patient 2 | TELO2 | 6 months | Behavior (1) + Attention (1) + Motor (1) |
| | Patient 3 | Micro under study | 9 months | Attention (1) + Fine motor skills (1) |
| | Patient 4 | Sjoëgren-Larsson | 9 months | Improved attention + Learning (1). |
| | | | | (*)Patient has spastic paraparesis, making it difficult to improve motor skills since there is long pathway dysfunction due to patient's illness |
| | Patient 5 | STXBP1 | 6 months | Improved behavior, attention (1) Patient had no previous motor problems |
| | Patient 6 | Secsep | >6 months | Improved behavior (1) + attention (2) + learning (1) + motor (0) |

| L-serine+5HTP+DHA | | | | |
|---|---|---|---|---|
| | Patient 7 | GNB1 | Esp. <3m | Improved behavior (2) + attention (2) + learning (2) + Motor (3) |

| Nicotinamide riboside+DHA | | | | |
|---|---|---|---|---|
| | | PLA2G6 | >9 months | Improved Motor (1) |
| | | PLA2G6 | >9 months | Improved Motor (1) |

The improvement observed in the patients, especially with regard to motor function, is much greater than that observed when L-serine was administered alone (trial carried out by the same research group published by David Soto et al.; in "L-Serine dietary supplementation is associated with clinical improvement of loss-of-function GRIN2B-related pediatric encephalopathy", Sci. Signal, 2019, vol. 12, pp. 1-15.

### Example 5: Evaluation of a formulation like that of Example 1 in patients

A formulation with the following composition (composition of the formula of Table 1 + spermidine) has been tested in 21 patients with different neurodevelopmental diseases (Rett syndrome, defects with hypoglutamatergic neurotransmission, mitochondrial function defects and others).

**Table 3: Formula:**

| | PATIENTS |
|---|---|
| Spermidine | 4 mg/20kg/day |
| Lecithin | 1000 mg/20kg/day |
| Choline | 500 mg/20kg/day |
| Phosphatidylcholine | 900 mg/20kg/day |
| Phosphatidylserine | 300 mg/20kg/day |
| N-acetylglucosamine | 900 mg/20kg/day |
| DHA | 90 mg/kg/day |
| EPA | 120 mg/kg/day |
| L-serine | 400 mg/kg/day |
| L-citrulline | 100 mg/kg/day |
| L-arginine | 100 mg/kg/day |
| L-tyrosine | 150 mg/kg/day |
| L-tryptophan | 100 mg/20kg/day |
| Uridine 5-monophosphate | 1000 mg/20kg/day |
| Sulforaphane | 8 mg/20kg/day |
| Nicotinamide riboside | 900 mg/20kg/day |
| Creatine | 300 mg/kg/day |
| Vitamin B₆ | 60 mg/20kg/day |
| Selenium | 0.1 mg/20kg/day |
| Magnesium | 30 mg/20kg/day |
| Zinc | 5 mg/20kg/day |

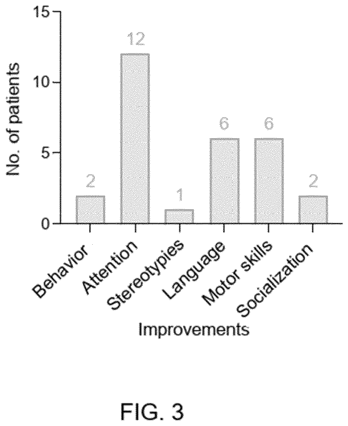

After one month with treatment at half the doses indicated in Table 1 of Example 1, improvements were observed in behavior (n= 2 patients), attention (12), improvement in stereotypies (1), language (6), motor skills (6) and socialization (2), as shown in Figure 3 and Table 4.

Improvements in behavior include responding to simple commands, behavior on a daily basis (assessed by people who interact with the patient, professionals and caregivers), activities like eating, sleeping, etc. It also includes greater interest in game dynamics, which in neuropediatric patients implies an improvement in cognitive and neurodevelopmental activity. Being interested in the game indicates improvement in verbal and abstract comprehension, interest in the environment, social cognition, visuospatial coordination and manual dexterity. This is only achieved with the activation of complex circuits that require good neuronal communication and myelination.

The improvements in attention are, with motor skills and language, the most reported. This has been reported both by professionals working with patients (reporting better attention in therapies such as music therapy or speech therapy), and by caregivers (describing patients as "more attentive", "more connected" and/or "more focused").

The increase in care for these patients is key to being able to work, furthermore, with other therapies. Furthermore, it is essential so that they can acquire other more complex learning. If they cannot connect with their environment, it is impossible for them to respond to therapies such as those included in early attention, reeducation of certain academic activities (reading, writing), logopedia, etc. In addition, it allows them to respond to simple commands (e.g., patient NH026 now responds by name and commands such as "turn off the light", which was previously unthinkable).

Stereotypies are repetitive, non-purposeful and uncontrolled movements that represent a great limitation in the patient's life. In some cases, such as the "hand washing movement" or "hand-mouth stereotypy" in Rett syndrome, are elements that characterize the disease. Currently there is no treatment available that reduces stereotypies.

Language development involves an advanced degree of neurological development. It requires the coordinated activation of numerous motor and attention areas and is only achieved when the brain has these circuits sufficiently prepared throughout neurodevelopment. In some patients like Rett patients, it is one of the first items to disappear, and many other patients never even develop it. Six patients have reported improvements in language with treatment. One of the patients had lost acquired language and after a month of treatment began to say words like "dad", "mom", "yes" and "no" with a clearly purposeful use, which represents an immense change in both the quality of life of the patient and that of their caregivers, as well as in patient management, who can now express some preferences. Other patients have started to formulate complete sentences (for example "I want the Tom and Jerry book" by patient NH022, which implies a high cognitive capacity.

Improvement in motor skills includes both fine motor skills (increased dexterity) and gross motor skills (e.g. when walking or going up and down stairs). This is related to the increase observed in the Rotarod test in mice (Example 3), in which motor coordination is measured, and is complemented with the data in the following table.

**Table 4:**

| Patient | Diagnosis | Reported improvements | | |
|---|---|---|---|---|
| | | Behavior | Attention | Stereot. |
| NH004 | Hypoglut. | x | X | |
| NH007 | Hypoglut. | x | X | |
| NH008 | Rett | | X | |
| NH010 | Rett | | X | X |
| NH015 | Rett | | | |
| NH016 | Rett | | X | |
| NH021 | Ph McD | | | |
| NH022 | Ph McD | | X | |
| NH023 | Ph McD | | X | |
| NH024 | Ph McD | | X | |
| NH025 | Ph McD | | | |
| NH026 | Ph McD | | X | |
| NH029 | Ph McD | | | |
| NH025 | Ph McD | | | |
| NH026 | Ph McD | | | |
| NH029 | Ph McD | | | |
| NH030 | Ph McD | | X | |
| NH031 | Rett | | | |
| NH032 | Rett | | | |
| NH035 | ND | | X | |
| NH036 | ND | | X | |

| Language | Motor skills | Socialization | | |
|---|---|---|---|---|
| NH004 | Hypoglut. | | | |
| NH007 | Hypoglut. | | | |
| NH008 | Rett | | | |
| NH010 | Rett | | | |
| NH015 | Rett | X | | |
| NH016 | Rett | | | |
| NH021 | Ph McD | X | X | |
| NH022 | Ph McD | X | X | |
| NH023 | Ph McD | | X | |
| NH024 | Ph McD | | X | |
| NH025 | Ph McD | X | | |
| NH026 | Ph McD | X | | |
| NH029 | Ph McD | | | |
| NH025 | Ph McD | | | |
| NH026 | Ph McD | | | |
| NH029 | Ph McD | | | X |
| NH030 | Ph McD | | | X |
| NH031 | Rett | X | X | |
| NH032 | Rett | | X | |
| NH035 | ND | | | |
| NH036 | ND | | | |

### CITATION LIST

### Patent literature

- WO2016083362A1
- WO2020/092616A1

### Non-patent literature

- David Soto et al. in "L-Serine dietary supplementation is associated with clinical improvement of loss-of-function GRIN2B-related pediatric encephalopathy", Sci. Signal, 2019, vol. 12, pp. 1-15
- Guy, J., Hendrich, B., Holmes, M., Martin, J. E. & Bird, A. A mouse Mecp2-null mutation causes neurological symptoms that mimic Rett syndrome. Nat Genet 27, 322-326 (2001).

## Claims

1. A nutraceutical combination comprising:
a) L-serine;
b) a uridine derivative selected from the group formed by uridine monophosphate, a uridine phosphate, a uridine acid halide with a carboxylic acid (C₂-C₂₀), a uridine ester with a carboxylic acid (C₂-C₂₀), and a nutraceutical or pharmaceutically acceptable uridine salt;
c) a compound selected from nicotinamide riboside, sulforaphane and mixtures thereof; and
d) a compound selected from the group consisting of lecithin, choline, phosphatidylcholine, phosphatidylserine, N-acetylglucosamine, at least one polyunsaturated fatty acid (C₂₀-C₂₂), and mixtures thereof.

2. The nutraceutical combination according to claim 1, wherein nicotinamide riboside and sulforaphane are present.

3. The nutraceutical combination according to any of claims 1-2, wherein the uridine derivative is uridine-5-monophosphate.

4. The nutraceutical combination according to any of claims 1-3, wherein the polyunsaturated fatty acid is selected from the group formed by docosahexaenoic acid (DHA), eicosapentaenoic acid (EPA), and mixtures thereof.

5. The nutraceutical combination according to any of claims 1-4, further comprising spermidine.

6. The nutraceutical combination according to any of claims 1-5, further comprising creatine and vitamin B₆.

7. The nutraceutical combination according to any of claims 1-6, further comprising an active ingredient selected from the group consisting of L-tyrosine, L-tryptophan, and mixtures thereof.

8. The nutraceutical combination according to any of claims 1-7, further comprising a compound selected from the group consisting of L-citrulline, L-arginine, and mixtures thereof.

9. The nutraceutical combination according to any of claims 1-8, further comprising a compound selected from the group consisting of selenium, magnesium, zinc, and mixtures thereof.

10. The nutraceutical combination according to any of claims 1-9, comprising an amount of each of the compounds that are present in the formulation suitable for administering, in one or more daily doses, a total daily amount of each of them as follows:
L-serine at a daily dose of 200 to 800 mg/kg/day;
uridine derivative at a daily dose of 500 to 2000 mg/20kg/day;
nicotinamide riboside at a daily dose of 450 to 1800 mg/20kg/day;
at least one polyunsaturated fatty acid (C₂₀-C₂₂) at a daily dose of 105 to 420 mg/kg/day;
sulforaphane at a daily dose of 4 mg/20kg/day to 16 mg/20kg/day;
lecithin at a daily dose of 500 to 2000 mg/20kg/day;
choline at a daily dose of 250 to 1000 mg/20kg/day,
phosphatidylcholine at a daily dose of 450 to 1800 mg/20kg/day;
phosphatidylserine at a daily dose of 150 to 600 mg/20kg/day; and
N-acetylglucosamine at a daily dose of 450 to 1800 mg/20kg/day.

11. A preparation in liquid or suspension form comprising the nutraceutical combination defined in any of claims 1-10.

12. The preparation according to claim 11, which is in a stick.

13. A preparation in powder form comprising a nutraceutical combination defined in any of claims 1-10, wherein the compounds thereof are solid.

14. A kit comprising two or more components, wherein each component comprises one or more of the compounds of the nutraceutical combination defined in any of claims 1-10,
wherein when the kit comprises one or more compounds in liquid form, they are either in separate liquid components or together in a single liquid component;
wherein the components of the nutraceutical combination are the following:
a) L-serine;
b) a uridine derivative selected from the group formed by uridine monophosphate, a uridine phosphate, a uridine acid halide with a carboxylic acid (C₂-C₂₀), a uridine ester with a carboxylic acid (C₂-C₂₀), and a nutraceutical or pharmaceutically acceptable uridine salt;
c) nicotinamide riboside, sulforaphane; or mixtures thereof;
d) a compound selected from the group consisting of lecithin, choline, phosphatidylcholine, phosphatidylserine, N-acetylglucosamine, at least one polyunsaturated fatty acid (C₂₀-C₂₂), and mixtures thereof; and
e) optionally one or more compounds selected from the group consisting of:
spermidine; creatine and vitamin B₆; an active ingredient selected from the group consisting of L-tyrosine, L-tryptophan, and mixtures thereof; a compound selected from the group consisting of L-citrulline, L-arginine, and mixtures thereof; and a compound selected from the group consisting of selenium, magnesium, zinc, and mixtures thereof; and
wherein the components that would be part, if present, of the liquid component are selected from the group consisting of polyunsaturated fatty acids, phosphatidylcholine and phosphatidylserine.

15. The kit according to claim 14, comprising:
a) a first component comprising the compounds of the combination that are solid; and
b) a second component comprising the compounds that are in liquid form.

16. A pharmaceutical product comprising a therapeutically effective amount of the nutraceutical combination defined in any of claims 1-10, optionally, with pharmaceutically acceptable excipients or carriers.

17. A powdered food product comprising the nutraceutical combination defined in any of claims 1-10, wherein the compounds thereof are solid.

18. The nutraceutical combination as defined in any of claims 1-10, for use in the treatment of a neurological disorder that is a neurodevelopmental disorder in children or adolescents or a neuropediatric condition.

19. The nutraceutical combination for use according to claim 18, wherein the treatment comprises improving cognitive functions and/or motor functions in children or adolescents who need it.

20. The nutraceutical combination for use according to any of claims 18-19, wherein the disorder is selected from the group consisting of Rett syndrome and grinpathies.
